(19) Europäisches Patentamt
European Patent Office
Office européen des brevets

(11) **EP 2 627 407 B1**

(12) **EUROPEAN PATENT SPECIFICATION**

(45) Date of publication and mention
of the grant of the patent:
**14.01.2015 Bulletin 2015/03**

(51) Int Cl.:
**A61N 5/06** *(2006.01)*    **A61B 5/04** *(2006.01)*
**G10L 17/00** *(2013.01)*

(21) Application number: **10858356.8**

(22) Date of filing: **13.10.2010**

(86) International application number:
**PCT/FI2010/050794**

(87) International publication number:
**WO 2012/049350 (19.04.2012 Gazette 2012/16)**

(54) **MODIFICATION OF PARAMETER VALUES OF OPTICAL TREATMENT APPARATUS**

VERÄNDERUNG VON PARAMETERWERTEN EINES OPTISCHEN BEHANDLUNGSGERÄTS

MODIFICATION DE VALEURS DE PARAMÈTRE D'UN APPAREIL DE TRAITEMENT OPTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**21.08.2013 Bulletin 2013/34**

(73) Proprietor: **Valkee Oy
90590 Oulu (FI)**

(72) Inventors:
• **NISSILÄ, Juuso
90590 Oulu (FI)**
• **AUNIO, Antti
90590 Oulu (FI)**

(74) Representative: **Kolster Oy Ab
Iso Roobertinkatu 23
PO Box 148
00121 Helsinki (FI)**

(56) References cited:
**WO-A1-2008/029001      WO-A1-2008/029001
GB-A- 2 333 962           JP-A- 2001 252 265
US-A- 5 562 719           US-A1- 2005 012 622
US-A1- 2005 075 532       US-A1- 2005 283 039
US-A1- 2007 083 079       US-A1- 2007 167 690
US-A1- 2007 192 038       US-A1- 2007 299 671
US-A1- 2008 119 994       US-A1- 2009 326 616
US-A1- 2010 010 371**

## Description

## Field

The invention relates generally to an apparatus for giving feedback and modifying the parameters of optical treatment.

## Background

Nowadays, people are applying various methods to improve their physical or mental condition. One may seek the improvement from traditional means, such as exercise or sleep, while another relies in more technical means, such as listening to music or receiving optical radiation. Common to the means is that the success of the method applied and the need to modify the treatment parameter values may not be reliably known afterwards.

Thus, it is important to provide a solution for giving feedback and to perform corrective actions accordingly.

## Brief description of the invention

Embodiments of the invention seek to improve the means for giving feedback on the optical treatment and modifying the optical treatment apparatus parameter values.

According to an aspect of the invention, there is provided an apparatus as specified in claim 1.

According to an aspect of the invention, there is provided a computer program product as specified in claim 15.

## List of drawings

In the following, the invention will be described in greater detail with reference to the embodiments and the accompanying drawings, in which

Figure 1 presents a solution for optical treatment according to an embodiment;

Figure 2 shows a solution for optical treatment according to an embodiment;

Figure 3 shows an apparatus for modifying the parameter values of the optical treatment apparatus according to an embodiment;

Figure 4 illustrates a possible indicator according to an embodiment;

Figure 5 illustrates an apparatus for determining indicators related to the speech of the user, according to an embodiment;

Figure 6 shows an exemplary activity curve during an observation period;

Figure 7 depicts an exemplary joint indicator curve during an observation period;

Figure 8 shows an exemplary solution for applying weighting coefficients;

Figure 9 illustrates some of the parameters of the optical treatment ; and

Figure 10 illustrates a method for modifying the parameter values of the optical treatment apparatus.

## Description of embodiments

The following embodiments are exemplary. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments.

Human nerve tissue includes regions that may be stimulated by optical radiation directed at the regions. Stimulation may have a metabolic and/or nervous response, which may appear as a change in alertness, diurnal (circadian) rhythm and in concentrations of several hormones and brain transmitters. The optical radiation may originate from nature or the optical radiation may have an artificial origin. A lack in the amount of the optical radiation energy (i.e. light) may cause a seasonal affective disorder (SAD) or depression in humans, for example.

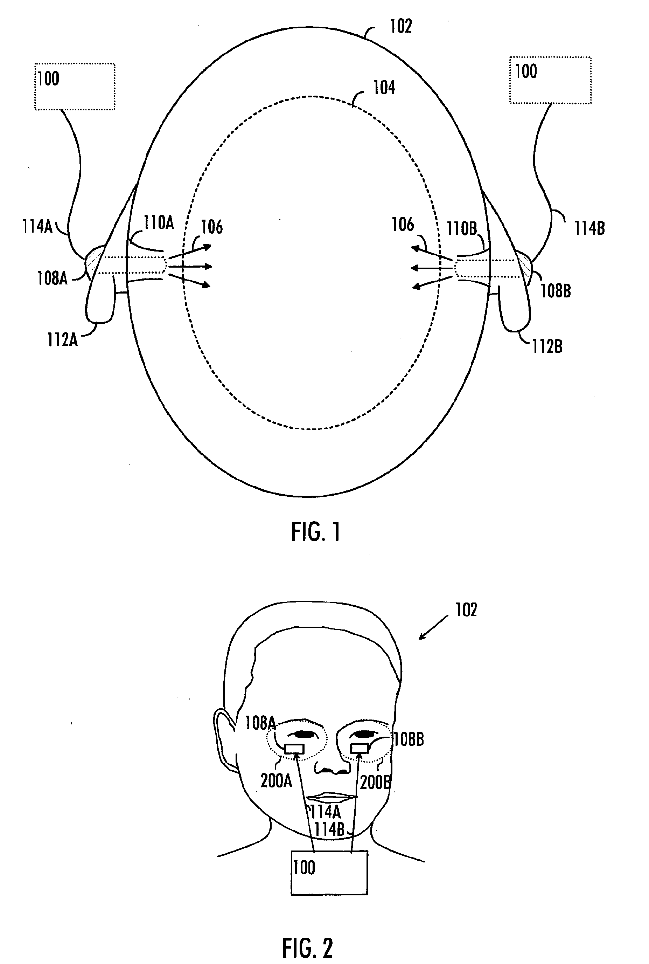

It may thus be advisable to use artificial optical radiation when natural light is not sufficient in order to prevent undesired physiological effects. This type of artificial light may be generated by bright light therapy devices installed in homes or workplaces, for instance. However, the optical treatment may also be obtained with a portable electronic device (PED) as shown in Figures 1 and 2. The portable electronic device may be carried by a user 102 without external support means. The portable electronic device may comprise radiation means for directing optical radiation energy non-invasively at a tissue of the user 102 in order to stimulate the user's 102 tissue. The PED 100 may comprise a central unit of the PED, radiation members 108A, 108B for emitting optical radiation and wires 114A and 114B for connecting the central unit and the radiation members 108A, 108B, respectively. The wires 114A and 114B may be used in conveying instructions to the radiation members 108A and 108B and/or to convey the optical energy to the radiation members 108A, 108B, respectively.

In an embodiment of figure 1, the user 102 is using the PED 100 such that the optical radiation 106 is directed through an external auditory canal 110A and 110B of the user 102 of the portable electronic device 100. The Figure shows the user's 102 head from top view. The external auditory canals 110A and 110B may for part of the user's 102 ears 112A and 112B. The radiation members 108A, 108B direct optical radiation 106 at the user's 102 external auditory canals 110A and 110B, respectively, which conveys the transmitted optical radiation energy 106 to the intracranial nerve tissue 104. In that case, the intracranial nerve tissue 104 is subjected to a treatment that has a response in the intracranial nerve tissue of a brain 104. In this context, the terms "optical radiation" and "optical radiation energy" are equivalent concepts, and the same reference number 106 is used to denote both. Optical radiation 106 typically comprises the wavelengths of infrared radiation, visible light and ultraviolet radiation. Propagation of the optical radiation energy 106 is based on the optical propagation of radiation 106 in tissue. When optical radiation energy 106 propagates in tissue, part of it is converted into heat. In addition, the wavelength distribution of optical radiation 106 typically changes due to absorption in tissue.

In an embodiment of Figure 2, the user 102 is receiving the optical radiation 106 extrapupillarily through the skin covering a user's 102 eye cavities 200A and 200B, thus resulting in diffuse propagation of optical radiation energy 106 to an optically sensitive tissue of the user's 102 eye. Thus, the user's 102 eye cavities 200A and 200B are exposed to optical radiation 106 which penetrates into the skin and enters the bone and soft tissue around the eye. A portion of the optical radiation energy 106 penetrates into the retina and reaches the photosensitive ganglion cells. A portion of the radiation energy propagates to the back of the eyeball and may reach the suprachiasmatic nucleus and the retinohypothalamic tract, for example.

Thus, the optical radiation energy 106 is received in the radiation-sensitive nerve tissue, which is stimulated by the optical radiation energy 106. For example, the intracranial nerve tissue of the brain 104 responsive to optical radiation energy 108 comprises, for example, cerebrum, cerebellum, vestibular organs, auditory organs, organs of smell, bulbus, a pineal body, also known as a pineal gland, and/or regions of autonomic regulation. The response may be based on a change in the concentration of melatonin hormone caused by the optical radiation 106, for example.

In an embodiment, the intracranial nerve tissue responsive to optical radiation 108 comprises a retina, whose ganglia cells may also sense light arriving from behind. Typically, the visual perception of ganglia cells is independent of seeing and not involved therein. Ganglia cells are in particular specialized for diffused light and their photosensitive pigment is melanopsin protein. When subjected to light, ganglia cells signal suprachiasmatic nucleus, which is the primary agent responsible for the circadian rhythm.

In an embodiment, the intracranial nerve tissue 102 responsive to optical radiation energy 108 comprises a suprachiasmatic nucleus (SCN) which regulates the pineal body, which back-regulates the SCN by excreting melatonin. The suprachiasmatic nucleus may also be responsible for controlling the circadian rhythms. The suprachiasmatic nucleus receives inputs from the photosensitive ganglion cells via the retinohypothalamic tract, which are illuminated in the embodiment of Figure 2.

It should be noted that the above-mentioned intracranial nerve tissues of brain 104 that are responsive to optical radiation energy 106 are only examples. Some of the light also affects through other means, for example through neuroendocrinology of diurnal rhythm. Intracranial nerve tissues, also in the cranial region, have several non-specific responses to optical radiation energy 106 and the temperature increase caused by the optical radiation energy 106. Such responses include increase in the metabolism of tissues and changes in the immune response.

The optical radiation 106 may affect the user 102 in many ways. The optical treatment that comprises illuminating an optically sensitive tissue of the user 102 with optical radiation energy 106 may improve physiological condition of the user 102. The effects which may be obtained with the optical treatment include but is not limited to the following: changes of diurnal rhythm, treatment of jetlag, treatment of sleep irregularity caused by shift work, exceptional change of sleep rhythm, treatment of seasonal affective disorder (SAD) and other affective syndromes, temporary increase of performance, waking up, alleviation of stress symptoms, nervous disorders caused by decreased light sensitivity of brain, improvement of plasticity of nerve system, and treatment of sexual insufficiency. It may further have effect in preventing an infection, treatment of certain diseases, such as the Parkinson's disease, treatment of depression, and treatment of depressive symptoms, for example.

For example, when a person 102 is suffering from a bipolar disorder or manic-depressive disorder, which is also referred to as bipolar affective disorder or manic depression, the person 102 may treat the disease with certain doses of illumination directed to photosensitive tissues of the person, such as the intracranial nerve tissue. The person 102 may notice that the depression is significantly relieved with appropriate doses of the optical radiation 106. It is however important to know whether the treatment is working or not and whether the parameter values, such as the appropriate dosage of illumination, of the optical treatment apparatus are to be modified or not.

For this reason, there is provided an apparatus and a method for giving feedback on the success of the optical treatment and for modifying the optical treatment apparatus parameter values. The optical treatment apparatus may be as in Figure 1 and 2, or it may be any apparatus providing optical radiation to the user, such as a lamp on the roof of the user's office, for example.

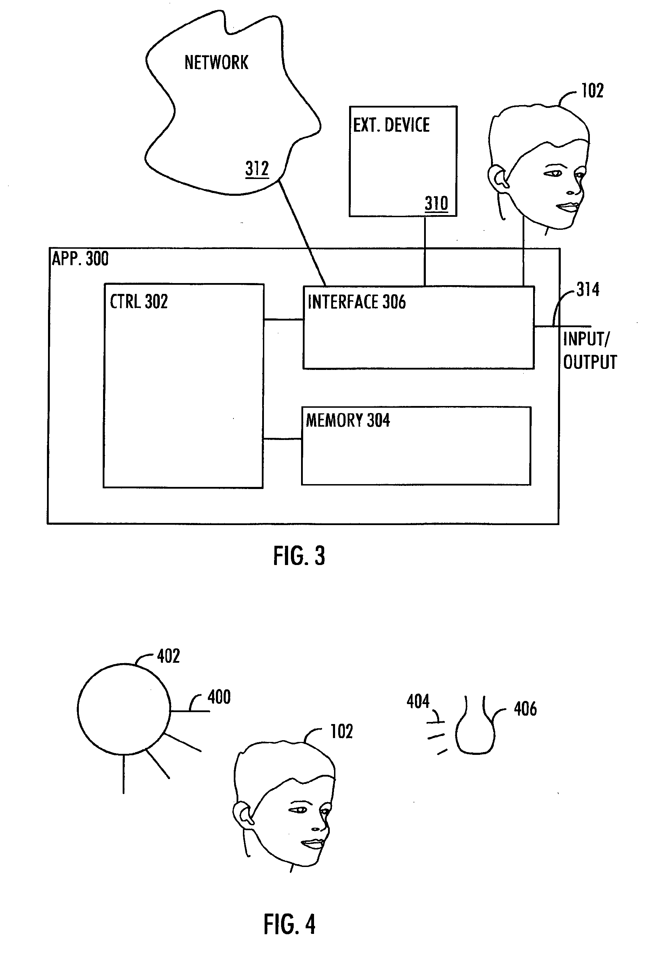

A very general architecture of an apparatus capable of giving feedback on the success of an optical treatment according to an embodiment is shown in Figure 3. Figure 3 shows only the elements and functional entities required for understanding the apparatus according to an embodiment. Other components have been omitted for reasons of simplicity. The implementation of the elements and functional entities may vary from that shown in Figure 3. The connections shown in Figure 3 are logical connections, and the actual physical connections may

5 **EP 2 627 407 B1** 6

be different. The connections can be direct or indirect and there can merely be a functional relationship between components. It is apparent to a person skilled in the art that the apparatus may also comprise other functions and structures.

When considering the apparatus of Figure 3, the apparatus 300 for giving feedback on the success of the optical treatment and for modifying the optical treatment parameter values may comprise an interface 306 for receiving first information related to parameters of a treatment for improving the mental or physical condition of at least one user. The treatment may be an optical treatment, wherein an optically sensitive tissue of the user is illuminated with optical radiation energy, as explained earlier. The information related to the parameters may comprise the current parameter values the user 102 has applied in the optical treatment. The apparatus 300 may then modify the current values if modification is seen appropriate.

Alternatively, the information related to the parameters may comprise the available parameters of the optical treatment. That is, parameters according to which the optical treatment takes place. This is advantageous so that if the user 102 has not used the optical treatment apparatus, such as the PED 100, previously or the PED 100 does not comprise predefined parameter values, the apparatus 300 may be used to define the parameter values for the first time, rather than modifying the existing parameter values.

The apparatus 300 may be comprised in a mobile phone, in a personal computer, in a palm computer, for example, or it may be a stand-alone separate apparatus. In an embodiment, the apparatus 300 is comprised in the optical treatment device, such as in the PED 100 of Figures 1 and 2.

The parameters, whose values may be set or modified, may be at least one of the following: at least one duration of illuminating the photosensitive nerve tissue of the user with the optical radiation energy, interval for illuminating the photosensitive nerve tissue of the user with the optical radiation energy, intensity of the optical radiation energy, and at least one point in time when the illumination takes place. The illumination may take place, for example, twice per day, once in the morning and once in the afternoon. Therefore, the obtained information may indicate when the at least one illumination has taken place, what was the intensity of each illumination (expressed in Lux or in Lumens, for example), and what was the duration of each illumination, for example. This way the apparatus 300 obtains knowledge of the parameters of the optical treatment.

The interface 306 may further receive second information comprising at least one indicator reflecting whether the optical treatment is successful or not, wherein the second information is different from the first information. In other words, the at least one indicator does not relate to the parameters of the optical treatment or to the values of the parameters of the optical treatment.

The at least one indicator may be seen as a consequence of the optical treatment. That is, as a result of a successful treatment the user 102 tends to do certain things or act in a certain way, and as a result of an unsuccessful treatment the user 102 tends to act in another way, as will be described later.

In an embodiment as shown in Figure 3, the at least one indicator is obtained in at least one of the following ways: inputted by the user 102, downloaded from an external electronic device 310, and downloaded from a network 312. Indicators may be received from all of these input means, or only from one of these input means, or from any combination of these input means, for example.

A given indicator may represent a value or characteristic related to the subject of the indicator for at least one predetermined observation period. The observation period may denote duration of time when information of the indicator is gathered, possibly stored and then communicated to the apparatus 300. The at least one predetermined observation period may be one hour, one day, one month, for example. The length of the period may depend on the physiological condition of the user 102 whose symptoms are attempted to be cured or alleviated with the optical treatment apparatus. If the condition is severe depression, the period may be selected longer than if the condition is a mere tiredness in the mornings, for example. This is because it may be assumed that alleviating a severe depression may take longer time than helping the user 102 with morning tiredness. The at least one predetermined observation period may also be such that only periods between, for example, 6 o'clock to 22 o'clock during several days are taken into account. The advantage is that the certain period of day may be discarded, such as the time when the user 102 is asleep.

In an embodiment, the at least one indicator comprises an indicator related to speech of the user 102 obtained through an external sensor worn by the user 102 during the at least one predetermined observation period. More specifically, the indicator related to the voice may be at least one of the following: the tone of the speech, the amount of laughter, the amount of aggressive speech voices, and the ratio between monologue and dialogue. The tonality of the speech voice may indicate happiness of mind or joyless mind and, thus, indicate how well the optical treatment for improving the physiological condition of the user 102 has worked because a happy mind may be seen as a result of a working optical treatment. Similarly, a high amount of laughter indicates successful treatment. Successful treatment may be indicated also by an increased social activity. The amount of social activities may be obtained when measuring the amount of dialogues, and the ratio of monologues and dialogues. A high amount of aggressive voices may on the other hand indicate unsuccessful treatment. In addition to the information obtained from the external device 310, the user 102 may input parameters to the apparatus 300 regarding the amount and type of speech during the

4

at least one predetermined observation period.

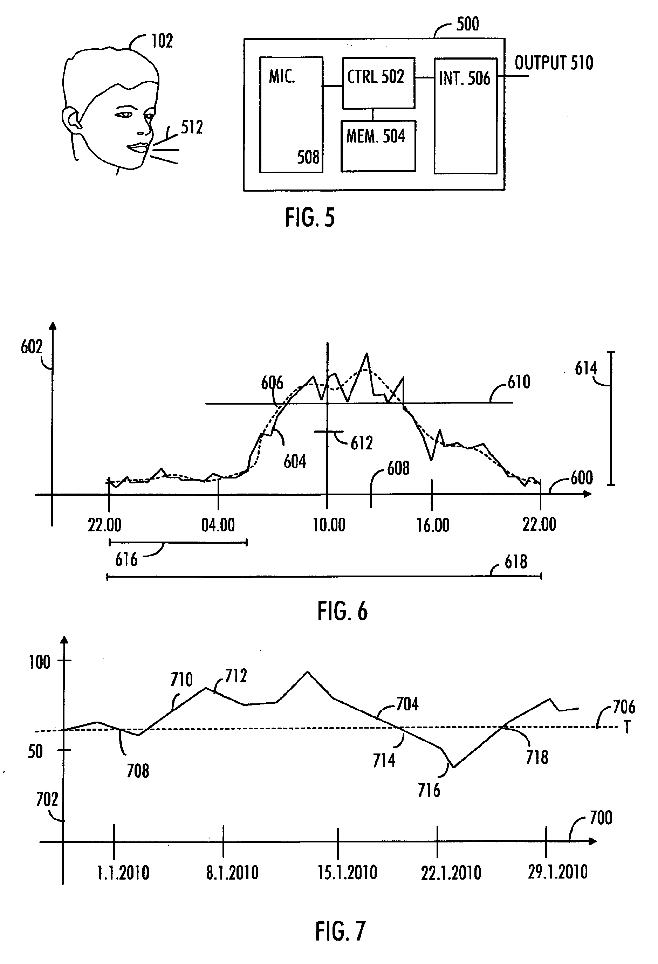

With reference to Figure 5, the indicator relating to the speech 512 may be obtained by the external sensor 500 worn by the user 102, wherein the external sensor 500 may take the place of the external device 310 in Figure 3. The external sensor 500 shown in Figure 5 may comprise a microphone 508 for listening and receiving the speech of the user 102. The speech voices listened may be processed at a processor 502. The processor 502 may, for example, differentiate aggressive voices from normal voices, discriminate between monologue and dialogue. In order to do this, the external sensor 500 may be taught to separate the user's voice from other persons' voices, and also taught what the normal tone of the voice of he user 102 is. The sensor 500 may also be equipped with a sensor that is in contact with user's 102 trachea in order to determine whether the received voice is spoken by the user 102, or detected from a person/radio/TV next to the user 102. The processor 502 may also determine the tone of the voice, and/or the amount of laughter in the voice. The apparatus 500 may also comprises a processor 504 for storing the information related to the voice or to store the voice samples for later processing. An interface 506 may be provided for conveying the processed information to the apparatus 300 of Figure 3, for example, via the output connection 510. The output connection 510 may be a wired or a wireless connection.

In order to perform the transmission from the external device 310, the interface 306 may be equipped with a connection to enable Bluetooth® communication, or any other suitable standard/non-standard wireless communication methods utilizing electric and/or magnetic fields. The interface 306 may be equipped with one or more antennas to receive the data from the external device 310, if needed.

Alternatively, or in addition to, the user 102 may input data to the apparatus 300 via inputting means such as a keyboard, a mouse, a microphone, etc.

In an embodiment, the at least one indicator comprises an indicator relating to the activity of the user 102. The indicator relating to activity may indicate at least one of the following during the at least one predetermined observation period: the amount of exercise performed by the user, the difference in magnitude between the average activity when asleep and the average activity when awake, the average activity, duration of activity above a certain threshold, amount of nocturnal activity, maximum activity level, the difference in magnitude between the highest activity level and the lowest activity level, and a circadian rhythm.

The amount of exercise may be recorded with the external device 310, such as a heart pulse meter, for example. The calories consumed may be recorded and the amount of exercise may be obtained from the stored calorie consumption value(s).

The average activity may be recorded with an activity meter being the external device 310 worn by the user 102. The data stored in the activity meter may then be uploaded to the apparatus 300 for further processing. The activity meter may record the intensity of the activity in METs (Metabolic Equivalent), which is assigned to each individual activity to indicate level of intensity of the specific activity. An activity with a 1 MET corresponds to the user's 102 resting metabolic rate, the rate at which the user 102 would burn calories when resting. Other activities are assigned typically higher MET values to indicate their intensity level relative to 1 MET. For example, playing football may produce a MET of 10. Thus, 10 times more calories are burned when playing football than sitting still. Alternatively to the activity meter, the MET values may be inputted by the user 102 by selecting the activity performed from a menu, wherein each activity has been assigned a suitable MET value beforehand. Although the MET is used here as an example, any term representing the user's activity suffices.

By obtaining knowledge of the activity during the at least one predetermined observation period, an exemplary activity curve as represented in Figure 6 may be obtained. The X-axis 600 represents a time line and the reference numeral 618 is used to denote the observation period. In this example, the observation period 618 is 24 hours, from 22.00 to 22.00. The Y-axis 602 may represent the activity in METS, in calories burned, in heart beats/minute, etc. The curve 604 represents the observed activity during the observation period 618. The curve 606 is a sliding average of the observed activity curve 604. Various indicators may be obtained from this type of activity representation.

Firstly, the amount of exercise performed by the user 102 may be determined by accumulating the activity 604 during the at least one observation period 618. In addition the maximum level of activity may be obtained. Moderate level of activity may indicate that the optical treatment is working as planned, whereas very low level of activity may be a symptom of a depression. As an example, an average activity level 612 of a person in depression during the observation period, expressed in METS for example, may be around 1,4 MET or lower. Thus, one object of the optical treatment may be to have the average activity level 612 to be 1,6 METS, for example. Accordingly, when lower average activity level than 1,6 is observed, the parameters of the optical treatment apparatus may be adjusted to reach the higher average activity level, for example. The desired level of activity may be individually set for each user 102 depending on the exercising habits of the person.

Secondly, the difference in magnitude between the average activity when asleep and the average activity when awake may be determined and the determined information may be applied for indicating the success of the optical treatment. A person without depression symptoms has typically such a contrast between the awake and the asleep periods that there are many activities when awake, but very little activities when asleep, thus a relatively high contrast. The contrast may be measured by knowing the activity curve 604/606 of the user 102. With low contrast, the optical treatment parameter values may be modified as the desired results have not been obtained. Also the difference in magnitude 614 between the highest activity level and the lowest activity level may be determined. This reveals the contrast of the activity of the user 102. Very low contrast indicates depression symptoms, for example, whereas very high contrast reflects mania-type symptoms. The appropriate, desired contrast which reflects wellbeing of the user 102, may be predetermined for each user 102.

Thirdly, the bed time 616 of the user 102 may be obtained by the activity sensor. The period having a low average activity typically represents the asleep period 616 of the user 102. An appropriate duration of the period 616 may indicate the successfulness of the treatment because a healthy person typically spends 6-8 hours asleep. For example, the activity sensor may determine the time when the user went asleep, the time when the user woke up, and the amount of nocturnal activity. A high nocturnal activity may indicate unsuccessful optical treatment. In addition the circadian rhythm or the average circadian rhythm may be obtained when the observation period is relatively long. A stable circadian rhythm may also indicate successful treatment.

Fourthly, the duration of activity 604/606 above a certain threshold 610 may be determined. This threshold 610 may be linked to the level that represents a typical exercise activity level of the user 102. This is advantageous so that the duration spent for exercising with certain intensity may be easily obtained and used as indication of the successfulness of the treatment. A person having no symptoms of depression typically exercises with intensity above the set level for a certain period of time during the predetermined observation period.

Fifthly, the point in time 608 for the highest activity level may be determined. A typical person without any symptoms of illness or disease may have predetermined the point in time when the highest activity usually takes place. Then, if it is noticed that the highest activity takes place during the asleep-period 616, it may imply that something is not correct. Thus, the treatment parameter values may need to be reconsidered.

In an embodiment, an external device 310 of Figure 3 may further measure a physiological parameter from the body of the user 102. A physiological parameter may characterize a body temperature, for example. The physiological parameter may then be provided to the apparatus 300 as one of the at least one indicator. A high body temperature may indicate unsuccessful treatment. This may imply that intense treatment is required as the high body temperature usually indicates an infection or alike.

In an embodiment, the at least one indicator comprises an indicator relating to at least one of the following during the at least one predetermined observation period: mood of the user, and the health of the user.

In order to measure the mood of the user 102, the external device 310 of Figure 3 may comprise a detector, such as a mechanical or optical movement sensing detector, for detecting the movement of the pupil of the eye of the user 102. When the pupil is relatively static throughout the observation period, it may be determined that the optical treatment is not working as planned, because a relatively static pupil may indicate symptoms of depression. However, excessive movement of the pupil may indicate manic symptoms which may be related to bipolar mental disorder, for example. Based on this information, the parameter values of the optical treatment may need to be modified. Alternatively, the mood of the user 102 may be given by the user 102 without any interaction with the external device 310.

The health of the user 102 may be enquired from the user 102 by using a questionary. The answers of which may be fed to the apparatus 300 by the user 102. The answers may be processed by the apparatus 300 in order to determine the success of the optical treatment. Thus, if the person provides improved health condition, the success of the treatment may be seen to exist, and vice versa.

In general, the questionary type of input method may be applied to any given indicator input.

In an embodiment, the at least one indicator comprises an indictor relating to at least one of the following: user's blood pressure, user's blood oxygen saturation, user's blood sugar level, user's encephalogram, user's skin electro conductivity, user's breathing frequency, user's eye movements, and user's limb movements.

Each of the indicators that are related to the user 102 may be linked to the previously recorded value of the same indicator. This is advantageous so that the trend of the indicator in question may be obtained and used as indication of a successful optical treatment. The target or desired level/value for each indicator may be predetermined taking into account the personal habits of the user 102. When comparing the desired level/value of the indicator to the trend of the indicator, the successfulness of the treatment may be determined. It should be noted thought that a person with semi-regular exercising habits, may not use the target levels of a high performance athlete who may exercise twice per day when no symptoms for depression exist. Thus, the desired target/level may be individually set for each user 102.

According to an embodiment, the apparatus of Figure 3 comprises a processor 302. The processor 302 may be implemented with a separate digital signal processor provided with suitable software embedded on a computer readable medium, or with a separate logic circuit, such as an application specific integrated circuit (ASIC). The processor 302 may comprise an interface, such as computer port, for providing communication capabilities. The processor 302 may be, for example, a dual-core processor or a multiple-core processor. The apparatus 300 may comprise a memory 304 connected to the processor 302. However, memory may also be integrated to the processor 302 and, thus, no memory 304 may be required.

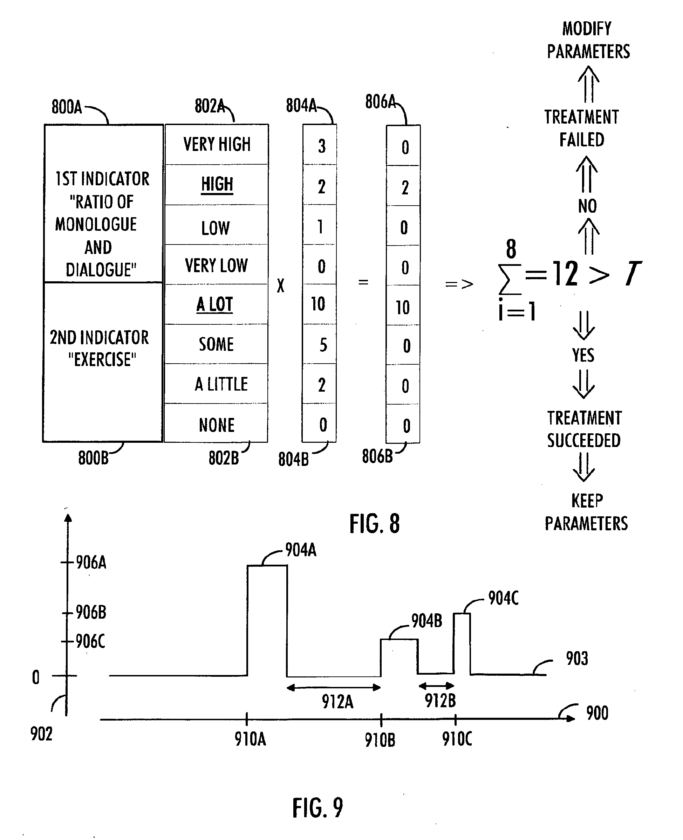

The processor 302 may apply an individual predetermined weighting coefficient for each of the received at least one indicator. Let us take as an example of a case when the user 102 inputs his/her indicator to the apparatus 300 in the form of answers to the questionary as shown in Figure 8. Each question in the questionary may have for example four predetermined answering possibilities, each given a predetermined weighting coefficient. However, it should be noted that the amount of answering possibilities may vary from one question to another, if certain type of question requires more or less available answering possibilities.

As shown in Figure 8, for a question 800A of "What is the ratio of monologues and dialogues?", possible answers 802A may include options "Very high", "High", "Low", and "Very low". Each answer may then be assigned a weighting coefficient 804A varying, for example, from 0 to 3 points. Similarly, the second question 800B in the questionary may be "How much exercise have you performed?". Possible answers 802B may be "A lot", "Some", "A little", and "None", as shown in Figure 8. Each of these answers are assigned with a weighting factor 804B, now varying from 0 to 10 as shown. The higher value in the coefficient may be seen to weight the importance of one parameter over the others (over another), for example. As can be seen from Figure 8, the selected answers may be multiplied by the weighting coefficients 804A and 804B in order to obtain the set of weighted values 806A and 806B. It may be understood that the selected answer is represented with "1" and the non-selected answer(s) is (are) represented with "0".

The processor 302 may then combine the weighted at least one indicator to one joint indicator value. In the example as shown in Figure 8, when the user 102 selects the bolded and underlined answers "High" and "A lot" for the questions 800A and 800B, respectively, the resulting coefficients are 2 and 10. The processor 302 may then combine these values into the joint indicator value, which results in this case to a value of 12. As shown, the combination of the weighted values (v) 806A and 806B may be a sum of the weighted values 806A and 806B. However, the combination of the weighted values 806A and 806B is not limited to summing, that is to

$$\sum_{i=1}^{n} v_i$$ , but the combination may be any arithmetic operation of the weighted values $V=[v_1, v_2, ..., v_n]$.

The predetermined value of the weighting coefficient may be based on the implication the indicator gives in relation to the successfulness or unsuccessfulness of the optical treatment. For example, when the given indicator reflects very successful treatment, a high or a low weighting coefficient value may be given. When the given indicator reflects very unsuccessful treatment, a low or a high weighting coefficient value may be given, respectively.

A person skilled in the art may learn which weighting coefficients values $v_i$ to use by testing different values for different indicators and selecting those values that seem to reflect the successfulness of the optical treatment in the most reliable way. In this learning period the actual physiological state of the user 102 may be analyzed so that the weighting coefficients may be set appropriately. For example, when the analysis reveals that the user is in an excellent physiological state (i.e., no depression exists), the answer(s) of at least one indicator given by the user 102 may be given a relatively high weighting coefficient. When the state of the user 102 is worse, the weighting coefficient for that answer of the same indicator is lower.

The processor 302 may then determine whether the parameter values of the optical treatment apparatus are to be modified or not, on the basis of the received first information and the joint indicator value. The first information may denote the current parameter values of the optical treatment, as described earlier. The result of the determination may be that the parameter values of the optical treatment apparatus are to be modified or that there is no need to modify the current parameter values. When there is no need to modify the parameter values, the processor 302 may further determine the optical treatment as successful. Thus, processor 302 may determine the success of the optical treatment on the basis of the received first information and the joint indicator value. When there is a need to modify the parameter values, the processor 302 may determine the optical treatment as unsuccessful.

In an embodiment, the processor may determine the optical treatment as successful when the joint indicator value exceeds at least one predetermined threshold T, as shown in Figure 8, in which case no modification of the parameter values is needed. That is, the joint indicator value is compared with the predetermined threshold T. It is further indicated in Figure 8 that in case of successful treatment there is no need to change the parameter values. When the threshold T is larger than the joint indicator value, the processor 302 may consider the treatment as unsuccessful and modify the parameter values.

The threshold T may be set individually for each user 102. The correct value may be learnt by trying different values for the thresholds and observing the user's physiological condition at the same time. When it is noticed that the physiological condition is not good, the parameter values of the optical treatment apparatus may be adjusted, the joint indicator value representing the non-adequate physiological condition may be calculated and the current threshold may be determined as a non-appropriate threshold value. On the other hand, when the physiological condition is at a sufficient level, the parameter values of the optical treatment apparatus may be kept unmodified, the joint indicator value representing the adequate physiological condition may be calculated and the current threshold may be determined as an appropriate threshold value. Thus the selected threshold value T may be used in the future so that the joint indicator value (representing the current physiological condition of the user 102) is compared against the selected threshold T. Amendments on the optical treatment values may be performed when the comparison result between the joint indicator and the threshold T so indicates.

In an embodiment, the processor 302 may analyze the currently determined joint indicator value such that the change from the previously determined joint indicator value is determined. The processor 302 may then determine the optical treatment as successful when the change from the previous joint indicator value is in the positive (desired) direction, in which case no modification of the parameter values may be needed. If the previously set joint indicator value is 10, for example, and the currently determined value is 12, then the processor 302 may keep the optical treatment parameter values unmodified as the trend is to the desired direction (in this case, the desired trend is increasing).

The processor 302 may observe the trend of the joint indicator value as well as the result of the comparison between the joint indicator value and the threshold T. Then, the processor 302 may determine whether to change the parameter values on the basis of both the trend and the comparison result. As an example, if the trend is increasing, but the target threshold is not reached, the processor 302 may determine to change the parameter values so that the target level may be obtained faster, for example. This may be achieved by increasing the dosage of the optical radiation, for example. On the other hand, if the threshold is reached but the trend is decreasing, the processor 302 may again decide to change the parameter values of the optical treatment apparatus so that the joint indicator value would not go below the threshold T.

This is shown in Figure 7 where a joint indicator curve 704, as observed over a period of one month (January 2010 on X-axis 700), is shown. The y-axis 702 shows the value of the joint indicator curve 704. As an example a range from 0 to 100 is selected. Threshold T is shown with a reference numeral 706. In point 708, the trend is decreasing and the joint indicator value is below the threshold 706, thus, the processor 302 decides to amend the parameter values of the optical treatment. The result is seen for example in point 710, where the trend is increasing and the joint indicator value is above the threshold 706, thus no changes needed. However, in point 712, the user experiences stress and the trend of the curve 704 becomes decreasing although the joint indicator value is still above the threshold. The processor may consequently decide to change the values of the optical treatment. After a while in point 714, the stress of the user becomes harder, and the curve 704 decreases below a threshold 706. Consequently, the processor 302 may decide that modification of the parameter values is needed. However, it may occur that the change did not effect the physiological condition of the user as planned as is represented by the joint indicator curve 704 having a steeper decrease in point 716. As a consequence, the processor 302 may further change the parameter values of the optical treatment. This time, the parameter values are modified to the correct direction so that the curve 704 becomes increasing and also above the threshold in point 718. This way the processor 302 may take the threshold 706 and the trend of the curve 704 into account when determining whether the parameter values need to be tuned or not.

The curve 704 may be seen as a "happy line" of the person. Alternatively only the threshold comparison or only the trend may be taken into account instead of both. The user 102 may himself determine how often the curve 704 is updated by inputting the indicators to the apparatus 300 with certain intervals.

Although the embodiment has been described so that indicators reflecting success are given higher weighting coefficients than indicators reflecting unsuccessful treatment, the opposite method is naturally possible. In an embodiment, there are x number of questions, each with y number of answer possibilities. Each answering possibility is weighted with a certain weighting coefficient within a range from zero to three. However, in this case three points for an indicator reflects a poor treatment and zero points for an indicator reflects a successful treatment. The value for a "happy line" may be set as value=$100-(J*100/(x*3))$, when the range for the happy line is limited between zero and one hundred, and $J$ is the sum of points, i.e., the joint indicator value of Figure 8. So if the user's 102 answers led to zero points for each indicator, the resulting happy line value is 100 and the treatment can be seen as highly successful.

Certain indicators may be given higher importance. For example, if the weighting coefficients for certain indicator inputs are within zero and three points, the weighting coefficient for certain other indicator inputs, such as for the feeling of the user 102, may be given a higher maximum weighting coefficient, such as four.

Certain indicator inputs may also be taken into account after the joint indicator value $J$ has been established. Those indicator inputs may then be given also negative weighting coefficients. For example, low amount of activity may decrease the calculated $J$ with three points, whereas high amount of activity may increase the J with 3 points. That is, in this case the weighting factor for the activity input would be between -3 and 3.

Similarly as in the example of Figure 8, the weighting coefficients may be given to each possible level/answer/option of each indicator. For example, when the indicator is the amount of laughter during the observation period, the predetermined weighting coefficient may be given to each range of laughter observed. When the speech sensor has detected 10 minutes of laughter during the observation period, the predetermined weighting coefficient may be 1, whereas when the speech sensor has detected 20 minutes of laughter during the observation period, the predetermined weighting coefficient may be 3, for example.

Similarly, each indicator that is inputted to the apparatus 300 affects the joint indicator value so that if the inputted indicator reflects successful treatment, the joint indicator value will be increased, whereas if the inputted indicator reflects unsuccessful treatment, the joint indicator value will be decreased. Of course when two given indicators reflect the opposite with regards to the successfulness of the treatment, the joint indicator value will either stay the same (if the weighting coefficients are the same for both inputs), increase (if the weighting coefficients for the indicator reflecting successful treatment is higher than the weighting coefficient for the indicator reflecting unsuccessful treatment), and decrease (if the weighting coefficients for the indicator reflecting unsuccessful treatment is higher than the weighting coefficient for the indicator reflecting successful treatment). Therefore, the joint indicator value reflects the total accumulated input and represents the successfulness of the optical treatment.

When the joint indicator value reflects a successful treatment, no modification of the parameter values may be needed. When the joint indicator value reflects an unsuccessful treatment, a modification of the parameter values may be needed in order to increase or decrease the effect of the optical treatment. The need to decrease the effect may exist when the user 102 is determined to have manic symptoms which may be related to bipolar mental disorder, for example. The need to increase the effect may exist when the user 102 is determined to have depression symptoms, for example. The means to increase or decrease the effect of the optical treatment will be described later.

In an embodiment, the interface 306 may receive third information comprising at least one factor affecting the successfulness of the optical treatment. The processor 302 may then take the third information into account when determining whether the parameter values of the optical treatment apparatus are to be modified or not.

In an embodiment, the at least one factor comprises a factor relating to the surrounding environment of the user 102 during the at least one predetermined observation period. In an embodiment, the third information may comprise a factor relating to the temperature of the environment. In an embodiment, the factor related to the surrounding environment of the user 102 may, as shown in Figure 4, comprise a magnitude of ambient light 400 or 404. The ambient light 400 coming from the sun 402 may be observed during the at least one predetermined observation period and be reported to the apparatus 300 of Figure 3 at the end of the observation period or in real time. Similarly, the ambient light 404 coming from a lamp 406 may be observed during the at least one predetermined observation period and be reported to the apparatus 300 of Figure 3. The ambient light 400 and/or 404 may affect to the person in the same way as the optical treatment given by the optical treatment device of Figure 1 and 2, for example. Thus, it may be of importance to know how much the user 102 experiences ambient light during the observation period as the magnitude of ambient light affects the successfulness of the optical treatment. The amount of ambient light may be accumulated so that one value for the amount of ambient light received during the observation period is obtained. This value may then be used in determining whether the parameter values of the optical treatment apparatus are to be modified or not. When the user 102 experiences sufficient amount of ambient light the dosage of illumination of the optical treatment may be reduced, and vice versa, for example.

The interface 306 may be used in such a way that the factor relating to the ambient light 400 and/or 404 is obtained from a personal database, wherein the personal database is generated by the user 102 and the personal database stores information relating to the amount of ambient light experienced during the at least one predetermined observation period. The magnitude may be given in Lux or in Lumens. The person may generate such personal database himself or he may wear an external device 310 which measures the amount of ambient light experienced. The database may be a combination of the above, so that the user 102 may discriminate between sun-light and artificial light whereas the external device 310 may determine the amount of the ambient light 400 and 404. The data may then be communicated to the apparatus 300 by the user 102 or transferred directly from the external device 310.

In an embodiment, the apparatus 300 may receive the indicator from the network 312. For this purpose the interface 306 may be equipped with a wireless or wired connection to the network 312, such as a local area network (LAN) or a wireless local area network (WLAN). This is advantageous so that the user does not have to update the information to the apparatus 300, but the apparatus 300 may itself collect the predetermined indicator from the network 312.

Alternatively, or in addition to, the user 102 may input the database to the apparatus 300 via inputting means such as a keyboard, a mouse, a microphone, etc.

As an example, the magnitude of ambient light may be obtained from the network 312 when the at least one location of the user 102 during the at least one predetermined observation period is known. Thus, the network 312 may be responsible of keeping an updated time-stamped weather related database for each location, such as for each city or village. When the location of the user 102 is known, the amount of the ambient light may be collected directly from the network 312 and, more specifically, from a weather database stored in the network. The location of the user 102 may be inputted by the user 102 himself, or downloaded from an external device keeping record of the locations of the user 102. Such an external device 310 may be a device capable of receiving information from the global positioning system (GPS), in other words, a GPS receiver. This is advantageous so that the apparatus 300 is aware of the prevailing weather condition (and the ambient light) in the location(s) of the user 102. Thus, the time of the year and the latitude of the location of the user 102 may be taken into account.

In an embodiment, the weather database in the network comprising the ambient light information may inform the user of the expected ambient light conditions in the current location. In this sense, the database works as a weather forecast for the user, wherein the forecast comprises information of the ambient light. Thus, in an embodiment, the factor related to the surrounding environment of the user 102 may comprise the expected magnitude of ambient light 400 during the next at least one observation period. The informing of the expected magnitude of ambient light may take place with a message to the user's mobile phone, the personal computer, etc. The information may also be directed directly to the apparatus 300. This enables the adjustment of the optical treatment apparatus parameters, such as when and how much optical radiation is to be taken from the optical radiation apparatus. The adjustment may take place so that the user adjusts the parameter values of the optical treatment apparatus, or so that the apparatus 300, without any interaction by the user, adjusts the parameter values. For example, if the user is in an area of high sun-light exposure, then the amount of the optical radiation taken from the optical treatment apparatus may be reduced, and vice versa. The weather forecast may be obtained from the network as described above, or inputted by the user. In this embodiment, the user 102 may provide the user's 102 expected whereabouts during the next at least one observation period.

In an embodiment, the at least one factor of the third information comprises a factor relating to a noise of the environment. This factor may affect the success of the treatment because noisy environments are generally not improving the user's physiological condition. Therefore, in a noisy environment, the amount of dosage from the optical treatment apparatus may be increased, the interval for taking the dosage may be reduced, for example.

In an embodiment, the at least one factor of the third information comprises a factor relating to at least one of the following during the at least one predetermined observation period: nutrition taken by the user 102, and medicine taken by the user 102. The nutrition consumed may be used to indicate the amount of fatty acids taken. Appropriate amount of fatty acids taken may boost the effects of the optical treatment to the right direction. Thus, it affects the successfulness of the optical treatment. By taking the amount of fatty acids consumed into account, the successfulness of the optical treatment may be more reliably determined. For example, if the user 102 has consumed a lot of fatty acids, such as the Omega fatty acids, and the treatment seems to be successful, part of the successfulness may be due to the appropriate eating habits and not entirely due to the optical treatment. Therefore it may be wise to verify the success of the treatment by further observation periods before making a decision whether to modify the optical treatment parameter values or not. This type of verification may be done also when the amount of ambient light is one factor of the third information. The nutrition consumed may be inputted by the user 102 himself. When the person has healthy eating habits, the dosage of the treatment may be decreased and vice versa, for example.

The medicines taken may also affect the success of the optical treatment in the same way as the proper nutrition taken. Thus, this information may be given by the user 102 to the apparatus 300.

In an embodiment, the third information represents a change in the location of the user. When the user 102 travels across time-zones the user 102 may need to adapt to a new circadian rhythm, for example. Moreover, the user 102 may suffer from a jet-lag which affects the successfulness of the treatment. As the apparatus 300 may receive the information relating to the change of the location of the user 102 during the at least one predetermined observation period, the parameter values of the optical treatment apparatus may be modified accordingly. For example, when the user 102 should to go to bed later due to the current time zone at the new location, the time of day when the last optical energy dosage is taken may be postponed, or the user 102 may take an additional dosage of the optical energy so that the user 102 may stay awake later.

Similarly as with the second information, the factors comprised in the third information may be weighted with predetermined weighting coefficients. Then a resulting joint factor value may be used similarly as the joint indicator value is used with references to Figure 7 and 8. In other words, the joint factor value may be compared to a predetermined threshold T2 (possibly different from T), or the trend of the joint factor value may be observed, or the trend and the comparison with the threshold $T_2$ may be simultaneously observed. Thus, the example, as shown in Figure 8, may be seen also as an example of how the predetermined weighting coefficients may be used to the third information. In this case, however, the 1st indicator and its possible answers or ranges/levels may be changed to a 1st factor and its possible answers, such as the weather having possible answers such as "Rainy", "Cloudy" "Semi-cloudy" and "Bright", and the 2nd indicator may be changed to a 2nd factor such as the medicine taken having possible answers such as "Anti depression medicine", "Head-ache medicine", "Caffeine tablets", for example. Alternatively, different medicine-related information may be inputted so that the amount of each taken medicine is obtained by the processor 302.

As said, the processor 302 may then take the third information into account when determining whether the parameter values of the optical treatment apparatus are to be modified or not. For example, when the user 102 has experienced a lot of ambient light the usage (effect) of the optical treatment may be decreased, and vice versa, for example.

It may also happen that one third information factor may imply to increase the dosage while another third information factor implies to decrease the usage of the optical treatment. In this case, the predetermined weighting factors may determine the value of the first factor more important than the inputted value of the second factor. This causes the joint factor value to change accordingly and the processor 302 to modify the parameter values in the direction as implied by the joint factor value and, therefore, as implied by the first inputted factor value. For example, when the first inputted factor reflects a successful treatment, a determined joint factor curve (similarly determined as the joint indicator curve 704) may have an increasing slope or it may be above the threshold $T_2$. As a result, the processor 302 may decide to keep the parameters unchanged.

When the second information implies to change the dosage so that the effect of the optical treatment is increased (increase the dosage and/or shorten the interval for taking the dosage) while the third information implies to decrease the effect (decrease the dosage and/or lengthen the interval for taking the dosage), the processor 302 may decide to give priority to the implication of the second information and to increase the effect of the optical treatment. This may be because, even though the user 102 has obtained a lot of ambient light and/or taken appropriate nutrition, for example, the previous setting of the treatment boosted with the third information factors has not been enough to reach the desired level of a successful optical treatment.

As said, the processor 302 may modify the parameter values of the optical treatment. The parameter values of the optical treatment apparatus that may be modified comprise at least one of the following with reference to Figure 9: at least one duration 904A to 904C for illuminating the user 102 with the optical radiation energy, at least one interval 912A to 912B for illuminating the user with the optical radiation energy, power of the optical radiation energy 906A to 906C, and at least one point in time 910A to 910C when the illumination takes place. In Figure 9, the x-axis 900 shows a time line of arbitrary length and the y-axis 902 shows the intensity of the illumination. The temporal distribution of the illumination, as shown with reference numeral 903, may be generated by modulating the electric power of the optical treatment device temporally. The modulation may be discrete, thus resulting in on and off states of the electric power or there may be a regulator for regulating the power for each individual dosage. The dosage may be seen to be the accumulated amount of optical radiation obtained in one illumination period.

By changing these parameter values, the processor 302 may increase or decrease the effect of the optical treatment. The increment may be done by increasing the dosage, by shortening the interval, by lengthening the duration, or by changing the time of point for giving the treatment. By changing the time of giving the treatment to earlier point in the morning, for example, the effect may be increased. Performing the opposite actions, the effect of the optical treatment may be decreased.

In an embodiment, a person skilled in the art may determine beforehand, for example by testing, how the parameter values of the optical treatment apparatus are to be modified when the joint indicator value is on the side of the threshold that reflects an unsuccessful treatment and/or when the joint indicator value reflects an undesired trend. The knowledge of how the parameter values are to be changed may comprise information on which parameters are to be changed, how much the selected parameter values are to be changed and to which direction the selected parameter values are to be changed.

When performing the tryouts, the physiological state of the user 102 may be determined on the basis of the second information given, and different modifications of the optical treatment may be tried. For each try, it may be analyzed whether the physiological condition is improved or not. If the physiological condition did improve, the previously given second information is recorded and the change of the parameter values that led to the change are also recorded as appropriate modification in relation to the given second information. If the physiological condition did not improve, the same takes place but now the modified parameter values are recorded as a non-appropriate modification relation to the given second information. This way the processor 302 may know how to modify the parameter values when the processor 302 obtains the second information.

In relation to the third information, similar tryouts may be performed in order to know what the appropriate modification is when the third information is known.

In an embodiment, there may be a set of parameter values from which sets the processor 302 selects one whenever modification is needed. The set may then change the current parameter values to correspond to the parameter values in the selected set. The sets may be so that one set is to be used when the trend of the joint indicator value is decreasing with a slope of - 10 degrees, while other set is used for a steeper slope, for example. Similarly, the difference to the threshold $T/T_2$ may be determined, and the set corresponding to the difference may be selected. The parameter values for each set may be determined by using the tryouts, for example.

The processor 302 may automatically without any user interaction perform the modification of the parameter values of the optical treatment apparatus. Alternatively, the processor 302 may output a question to the user 102. The question may ask the user 102 whether the user 102 accepts the proposed modifications. The user 102 may then either accept or decline the proposed changes. The question may be outputted through a display comprised in the apparatus 300, or via an external output means which may be connected to the apparatus 300 via an input/output connection 314. Alternatively, the processor 302 may instruct the user to modify the parameter values in a way as shown in the output means.

The output connection 314 of Figure 3 may be used in showing the user 102 that modifications have been performed and indicate what the modifications were.

The time instance of each modification and the type of each modification may be visually shown to the user 102 in a single view together with the joint indicator curve and/or the joint factor curve. This allows the user 102 to easily see how the modification has affected the physiological state of the user 102 over the period for which the information is shown.

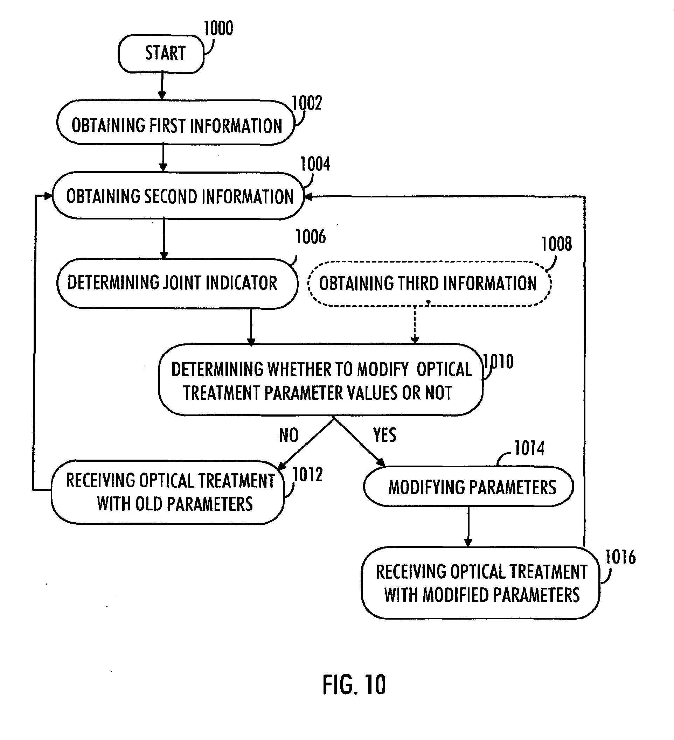

Figure 10 describes a method for modifying parameter values of an optical treatment. The method starts in step 1000. The method comprises in step 1002 obtaining the first information related to the parameters of a treatment for improving the mental or physical condition of a user, and in step 1004 obtaining second information comprising at least one indicator indicating whether the optical treatment is successful or not, wherein the second information is different from the first information. In step 1006 the method comprises applying a weighting coefficient for each of the at least one indicator, and combining the weighted at least one indicator to a joint indicator value. In addition the method may comprise in step 1008 obtaining the third information comprising at least one factor affecting the successfulness of the optical treatment, wherein the third information is different from the first information and from the second information. In step 1010, the method may determine whether the parameter values of the optical treatment apparatus are to be modified or not, on the basis of the received first information and the received second information. In addition the third information may be taken into account when performing the determination. If the determination result indicates that parameter values need not be modified, the method proceeds to step 1012 where the user 102 receives the optical treatment with existing (old) parameter values. After an observation period, the method restarts from step 1004 onwards, and the need to modify the parameter values is re-determined. If on the other hand, the determination result in step 1010 indicates that optical treatment parameter values are to be modified, the method proceeds to step 1014 where the parameter values are modified on the basis of the obtained information, as explained earlier. In step 1016 the user 102 receives the optical treatment with the modified parameter values. After an observation period, the method restarts from step 1004 onwards, and the need to modify the parameter values further is re-determined.

Even thought the method and the apparatus have been described in view of a single user, the method and the apparatus may be applied with respect to a plurality of users 102. The plurality of users 102 may be present, when, for example, a number of users enjoy the optical treatment. Then the need to modify the parameter values for the plurality of users 102 and the successfulness of the optical treatment for the plurality of users 102 are determined.

The modification may take place individually meaning that the modification is performed individually for each user according to the first, the second, and possibly, the third information related him/her. Alternatively, the modification may take place such that the first, the second, and possibly, the third information related each user is determined and obtained. Then, a common joint indicator value and, possibly, a common joint factor value are determined. The common joint indicator value and the common joint factor value may be obtained as an average of the individual joint indicator values and as an average of the individual joint factor values, respectively. Alternatively, the weighting factors for the given indicators/factors related to every user 102 may be all combined into one value without any averaging taking place. The decision to modify the parameters and the determination of the successfulness of the treatment may then be based on the common joint indicator value and the common joint factor value. Thus, the same modifications, if any, are performed for each of the users in the plurality of users.

The techniques and methods described herein may be implemented by various means. For example, these techniques may be implemented in hardware (one or more devices), firmware (one or more devices), software (one or more modules), or combinations thereof. For a hardware implementation, the apparatus of Figure 3 may be implemented within one or more application-specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, other electronic units designed to perform the functions described herein, or a combination thereof. For firmware or software, the implementation can be carried out through modules of at least one chip set (e.g. procedures, functions, and so on) that perform the functions described herein. The software codes may be stored in a memory unit and executed by processors. The memory unit may be implemented within the processor or externally to the processor. In the latter case, it can be communicatively coupled to the processor via various means, as is known in the art. Additionally, the components of the systems described herein may be rearranged and/or complemented by additional components in order to facilitate the achievements of the various aspects, etc., described with regard thereto, and they are not limited to the precise configurations set forth in the given figures, as will be appreciated by one skilled in the art.

Thus, according to an embodiment, the apparatus may comprise means for performing the tasks of Figures 3 to 10. More specifically, the apparatus may comprise interfacing means for receiving first information related to the parameters of a treatment for improving the mental or physical condition of a user, wherein the treatment is an optical treatment according to which an optically sensitive tissue of the user is illuminated with optical radiation energy. The apparatus may further comprise interfacing means for receiving second information comprising at least one indicator whether the optical treatment is successful or not, wherein the second information is different from the first information. The apparatus may further comprise processing means for applying at least one predetermined weighting coefficient to each of the received at least one indicator, processing means for combining the weighted at least one indicator to a joint indicator value, and processing means for determining whether the parameter values of the optical treatment apparatus are to be modified or not, on the basis of the received first information and the received second information.

Embodiments of the invention may be implemented as computer programs in the apparatus 300 according to the embodiments. The computer programs comprise instructions for executing a computer process for determining whether to modify he optical treatment parameter values or not. The computer program implemented in the apparatus 300 may carry out, but is not limited to, the tasks related to Figures 3 to 10.

The computer program may be stored on a computer program distribution medium readable by a computer or a processor. The computer program medium may be, for example but not limited to, an electric, magnetic, optical, infrared or semiconductor system, device or transmission medium. The computer program medium may include at least one of the following media: a computer readable medium, a program storage medium, a record medium, a computer readable memory, a random access memory, an erasable programmable read-only memory, a computer readable software distribution package, a computer readable signal, a computer readable telecommunications signal, computer readable printed matter, and a computer readable compressed software package.

Even though the invention has been described above with reference to an example according to the accompanying drawings, it is clear that the invention is not restricted thereto but can be modified in several ways within the scope of the appended claims. Further, it is clear to a person skilled in the art that the described embodiments may, but are not required to, be combined with other embodiments in various ways.

## Claims

1. An apparatus for optical treatments, comprising:

   an interface (306) configured to receive first information related to the parameters of a treatment for improving the mental or physical condition of at least one user (102);
   radiation members (108A, 108B) configured to be carried by the user (102) in the ears of the user (102) and deliver optical radiation energy (106) to illuminate an optically sensitive tissue (104) of the user (102) directly through an external auditory canal (110A and 110B) of the user (102) for curing or alleviating a predetermined disease;
   wherein the interface (306) is further configured to receive second information comprising at least two different indicators (800A, 800B) indicating whether the optical treatment is successful or not, wherein the second information is different from the first information;
   and the apparatus (300) further comprises a processor (302) configured to:
   apply an individual predetermined weighting coefficient (804A, 804B) to each of the at least two indicators (800A, 800B) received, wherein the value of a given weighting coefficient is based on the implication the indicator gives in relation to the successfulness or unsuccessfulness of the optical treatment in curing or alleviating the predetermined disease;
   combine the weighted at least two indicators to a joint indicator value, wherein the joint indictor value represents the successfulness or unsuccessfulness of the optical treatment in curing or alleviating the predetermined disease; and
   determine whether the parameter values of the optical treatment apparatus are to be modified or not, on the basis of the received first information and the joint indicator value.

2. The apparatus (300) of claim 1, wherein the processor (302) is further configured to:

   analyze the currently determined joint indicator value such that the change from the previously determined joint indicator value is determined, and determine the optical treatment as successful when the change from the previous joint indicator value is in the positive direction, in which case no modification of the parameter values is needed, or
   determine the optical treatment as successful when the joint indicator value exceeds at least one predetermined threshold, in which case no modification of the parameter values is needed.

3. The apparatus (300) of any of claims 1 to 2, wherein the at least two indicators are obtained in at least one of the following ways: inputted by the user (102), downloaded from an external electronic device (310), and downloaded from a network (312).

4. The apparatus (300) of any of claims 1 to 3, wherein at least one indicator (800A, 800B) of the at least two indicators comprises an indicator relating to voice of the user (102) obtained through an external sensor worn by a user (102) during the at least one predetermined observation period.

5. The apparatus (300) of claim 4, wherein the indicator relating to the speech is at least one of the following: the tone of the speech, the amount of laughter, the amount of aggressive speech, and the ratio between monologue and dialogue.

6. The apparatus (300) of any of claims 1 to 5, wherein at least one indicator (800A, 800B) of the at least two indicators comprises an indicator relating to activity of the user (102) and indicates at least one of the following during the at least one predetermined observation period: the amount of exercise performed by a user (102), the difference in magnitude between the average activity when asleep and the average activity when awake, the average activity (612), duration of activity above a certain threshold, amount of nocturnal activity, maximum activity level, a circadian rhythm, the difference in magnitude between the highest activity level and the lowest activity level (614), and duration of asleep (616).

7. The apparatus (300) of any of claims 1 to 6, wherein the interface (306) is further configured to:

   receive third information comprising at least one factor affecting the successfulness of the optical treatment, wherein the third information is different from the first information and from the second information; and
   the processor (302) is further configured to take the third information into account when determining whether the parameter values of the optical treatment apparatus are to be modified or not.

8. The apparatus (300) of claim 7, wherein the at least one factor comprises a factor relating to the surrounding environment of the user (102) during at least one predetermined observation period.

9. The apparatus (300) of any of claims 7 to 8, wherein the factor relates to magnitude of ambient light (400, 404), and the interface (306) is further configured to:

   receive the magnitude of ambient light (400, 404) from the network (312) when the at least one location of the user (102) during the at least one predetermined observation period is known, wherein the network (312) maintains an updated time-stamped weather related database for each location.

10. The apparatus (300) of any of claims 7 to 8, wherein the factor relates to magnitude of ambient light (400, 404), and the interface (306) is further configured to:

   obtain the magnitude of ambient light (400, 404) from a personal database, wherein the personal database is generated by the user (102) and stores information relating to the amount of ambient light (400, 404) experienced during the at least one predetermined observation period.

11. The apparatus (300) of any of claims 7 to 10, wherein the at least one factor comprises a factor relating to at least one of the following during the at least one predetermined observation period: nutrition taken by the user (102), and medicine taken by the user (102).

12. The apparatus (300) of any of claims 7 to 11, wherein the processor (302) is further configured to:

   prioritize the implication of the second information over the implication of the third information, wherein the second and the third information imply the opposite with regards to modification of the parameter values of the optical treatment apparatus.

13. The apparatus (300) of any of claims 1 to 12, wherein the processor (302) is further configured to:

   modify, or instruct the user (102) to modify, the parameter values of the optical treatment apparatus on the basis of the determination result, wherein the value of at least one of the following parameters is modified: at least one duration (904) for illuminating the user (102) with the optical radiation energy (106), interval (912) for illuminating the user (102) with the optical radiation energy (106), power (906) of the optical radiation energy (106), and at least one point in time (910) when the illumination takes place.

14. The apparatus (300) of any of claims 1 to 13, wherein at least one indicator (800A, 800B) of the at least two indicators comprises an indicator indicating the amount of exercise performed by a user (102) during the at least one predetermined observation period.

15. A computer program product embodied on a distribution medium readable by a computer and comprising program instructions which, when loaded into an apparatus of any of claims 1 to 14, cause the apparatus to execute the following:

   obtaining first information related to the parameters of a treatment for improving the mental or physical condition of at least one user (102);
   obtaining second information comprising at least two different indicators (800A, 800B) indicating whether the optical treatment is successful or not, wherein the second information is different from the first information;

applying an individual predetermined weighting coefficient (804A, 804B) to each of the at least two indicators (800A, 800B) received, wherein the value of a given predetermined weighting coefficient is based on the implication the indicator gives in relation to the successfulness or unsuccessfulness of the optical treatment;

combining the weighted at least two indicators to a joint indicator value, wherein the joint indictor value represents the successfulness or unsuccessfulness of the optical treatment in curing or alleviating the predetermined disease; and

determining whether the parameter values of the optical treatment apparatus are to be modified or not, on the basis of the received first information and the joint indicator value.


**Patentansprüche**

1. Eine Vorrichtung zur optischen Behandlung, umfassend:

eine Schnittstelle (306), die konfiguriert ist, um eine erste Information, die sich auf die Parameter einer Behandlung zur Verbesserung des mentalen oder physischen Zustands von mindestens einem Anwender (102) bezieht, zu empfangen;

und Strahlungsmittel (108A, 108B), die konfiguriert sind, um von dem Anwender (102) in den Ohren des Anwenders (102) getragen zu werden und optische Strahlungsenergie (106) zu liefern, um ein optisch sensitives Gewebe (104) des Anwenders (102) direkt durch einen äußeren Gehörgang (110A und 110B) des Anwenders (102) zu beleuchten zur Heilung oder Linderung einer vorbestimmten Krankheit;

wobei die Schnittstelle (306) weiter konfiguriert ist, um eine zweite Information zu empfangen, die mindestens zwei verschiedene Indikatoren (800A, 800B) umfasst, die anzeigen, ob die optische Behandlung erfolgreich ist oder nicht, wobei sich die zweite Information von der ersten Information unterscheidet; und wobei die Vorrichtung (300) weiter einen Prozessor (302) umfasst, der konfiguriert ist, um:

einen individuell vorbestimmten Gewichtungskoeffizienten (804A, 804B) auf jeden der mindestens zwei empfangenen Indikatoren (800A, 800B) anzuwenden, wobei der Wert eines vorgegebenen Gewichtungskoeffizienten auf der Implikation basiert, die der Indikator in Bezug auf den Erfolg oder die Erfolglosigkeit der optischen Behandlung bei der Heilung oder Linderung der vorbestimmten Krankheit gibt;

die gewichteten mindestens zwei Indikatoren zu einem gemeinsamen Indikatorwert zu kombinieren, wobei der gemeinsame Indikatorwert den Erfolg oder die Erfolglosigkeit der optischen Behandlung zur Heilung oder Linderung der vorbestimmten Krankheit darstellt; und

zu bestimmen, ob die Parameterwerte der optischen Behandlungsvorrichtung geändert werden müssen oder nicht, auf Basis der empfangenen ersten Information und des gemeinsamen Indikatorwerts.

2. Die Vorrichtung (300) nach Anspruch 1, wobei der Prozessor (302) weiter konfiguriert ist, um:

den aktuell bestimmten gemeinsamen Indikatorwert zu analysieren, sodass die Änderung von dem zuvor bestimmten Indikatorwert ermittelt wird, und die optische Behandlung als erfolgreich zu bestimmen, wenn die Änderung von dem vorherigen gemeinsamen Indikatorwert in der positiven Richtung liegt, in welchem Fall keine Änderung der Parameterwerte nötig ist, oder die optische Behandlung als erfolgreich zu bestimmen, wenn der gemeinsame Indikatorwert mindestens einen vorbestimmten Schwellenwert überschreitet, in welchem Fall keine Änderung der Parameterwerte nötig ist.

3. Die Vorrichtung (300) nach einem der Ansprüche 1 bis 2, wobei die mindestens zwei Indikatoren auf mindestens eine der folgenden Weisen erhalten werden:

eingegeben durch den Anwender (102), heruntergeladen von einem externen elektronischen Gerät (310) und heruntergeladen aus einem Netzwerk (312).

4. Die Vorrichtung (300) nach einem der Ansprüche 1 bis 3, wobei mindestens ein Indikator (800A, 800B) der mindestens zwei Indikatoren einen Indikator umfasst, der sich auf die Stimme des Anwenders (102) bezieht, erhalten durch einen externen von einem Anwender (102) getragenen Sensor während der mindestens einen vorbestimmten Beobachtungsperiode.

5. Die Vorrichtung (300) nach Anspruch 4, wobei der Indikator, der sich auf die Sprechweise bezieht, mindestens einer der Folgenden ist: der Tonfall der Sprechweise, die Menge des Lachens, die Menge der aggressiven Sprechweise, und das Verhältnis zwischen Monolog und Dialog.

**6.** Die Vorrichtung (300) nach einem der Ansprüche 1 bis 5, wobei mindestens ein Indikator (800A, 800B) der mindestens zwei Indikatoren einen Indikator umfasst, der sich auf die Aktivität des Anwenders (102) bezieht, und mindestens eins von Folgendem während der mindestens einen vorbestimmten Beobachtungsperiode anzeigt: die Menge der durch den Anwender (102) durchgeführten Übungen, den Größenunterschied zwischen der durchschnittlichen Aktivität im Schlafzustand und der durchschnittlichen Aktivität im Wachzustand, die durchschnittliche Aktivität (612), die Aktivitätsdauer oberhalb eines gewissen Schwellenwerts, die Menge der nächtlichen Aktivität, das maximale Aktivitätslevel, einen zirkadianen Rhythmus, den Größenunterschied zwischen dem höchsten Aktivitätslevel und dem niedrigsten Aktivitätslevel (614) und die Dauer des Schlafzustands (616).

**7.** Die Vorrichtung (300) nach einem der Ansprüche 1 bis 6, wobei die Schnittstelle (306) weiter konfiguriert ist, um:

eine dritte Information zu empfangen, umfassend mindestens einen Faktor, der den Erfolg der optischen Behandlung beeinflusst, wobei sich die dritte Information von der ersten Information und von der zweiten Information unterscheidet; und
wobei der Prozessor (302) weiter konfiguriert ist, um die dritte Information zu berücksichtigen, wenn bestimmt wird, ob die Parameterwerte des optischen Behandlungsgeräts geändert werden müssen oder nicht.

**8.** Die Vorrichtung (300) nach Anspruch 7, wobei der mindestens eine Faktor einen Faktor umfasst, der sich auf die umliegende Umwelt des Anwenders (102) während mindestens einer vorbestimmten Beobachtungsperiode bezieht.

**9.** Die Vorrichtung (300) nach einem der Ansprüche 7 bis 8, wobei sich der Faktor auf die Stärke des Umgebungslichts (400,404) bezieht und die Schnittstelle (306) weiter konfiguriert ist, um:

die Stärke des Umgebungslichts (400,404) aus dem Netzwerk (312) zu empfangen, wenn der mindestens eine Ort des Anwenders (102) während der mindestens einen vorbestimmten Beobachtungsperiode bekannt ist, wobei das Netzwerk (312) eine aktualisierte zeitgestempelte wetterbezogene Datenbank für jeden Ort führt.

**10.** Die Vorrichtung (300) nach einem der Ansprüche 7 bis 8, wobei sich der Faktor auf die Stärke des Umgebungslichts (400,404) bezieht und die Schnittstelle (306) weiter konfiguriert ist, um:

die Stärke des Umgebungslichts (400,404) von einer persönlichen Datenbank zu empfangen, wobei die persönliche Datenbank durch den Anwender (102) generiert wird und Informationen speichert, die sich auf die Stärke des Umgebungslichts (400,404) beziehen, die während der mindestens einen vorbestimmten Beobachtungsperiode erfahren wurde.

**11.** Die Vorrichtung (300) nach einem der Ansprüche 7 bis 10, wobei der mindestens eine Faktor einen Faktor umfasst, der sich auf mindestens eins von Folgendem während der mindestens einen vorbestimmten Beobachtungsperiode bezieht:

Nahrung, die durch den Anwender (102) aufgenommen wurde, und Arzneimittel, das durch den Anwender (102) aufgenommen wurde.

**12.** Die Vorrichtung (300) nach einem der Ansprüche 7 bis 11, wobei der Prozessor (302) weiter konfiguriert ist, um:

der Implikation der zweiten Information Vorrang vor der Implikation der dritten Information zu geben, wobei die zweite und die dritte Information Gegenteiliges bezüglich der Änderung der Parameterwerte des optischen Behandlungsgeräts implizieren.

**13.** Die Vorrichtung (300) nach einem der Ansprüche 1 bis 12, wobei der Prozessor weiter konfiguriert ist, um:

die Parameterwerte des optischen Behandlungsgeräts auf Basis des Bestimmungsergebnisses zu ändern oder den Anwender (102) zu instruieren, diese zu ändern, wobei der Wert von mindestens einem der folgenden Parameter geändert wird: mindestens eine Dauer (904) zur Beleuchtung des Anwenders (102) mit der optischen Strahlungsenergie (106), Intervall (912) zur Beleuchtung des Anwenders (102) mit der optischen Strahlungsenergie (106), Leistung (906) der optischen Strahlungsenergie (106) und mindestens ein Zeitpunkt (910), wann die Beleuchtung stattfindet.

**14.** Die Vorrichtung (300) nach einem der Ansprüche 1 bis 13, wobei mindestens ein Indikator (800A, 800B) der mindestens zwei Indikatoren einen Indikator umfasst, der die Menge der durch den Anwender (102) durchgeführten Übungen während der mindestens einen vorbestimmten Beobachtungsperiode anzeigt.

**15.** Ein Computerprogrammprodukt verkörpert auf einem Verbreitungsmedium, das von einem Computer lesbar ist und Programmanweisungen umfasst, die, wenn sie auf eine Vorrichtung nach einem der Ansprüche 1 bis 14 geladen werden, die Vorrichtung dazu veranlassen, das Folgende auszuführen:

Erhalten einer ersten Information, die sich auf die Parameter einer Behandlung zur Verbesserung des mentalen oder physischen Zustands von mindestens einem Anwender (102) bezieht;
Erhalten einer zweiten Information, die mindestens zwei unterschiedliche Indikatoren (800A, 800B) umfasst, die anzeigen, ob die optische Behandlung erfolgreich ist oder nicht, wobei sich die zweite Information von der ersten Information unterscheidet;
Anwenden eines individuell vorbestimmten Gewichtungskoeffizienten (804A, 804B) auf jeden der mindestens zwei empfangenen Indikatoren (800A, 800B),
wobei der Wert eines gegebenen vorbestimmten Gewichtungskoeffizienten auf der Implikation basiert, die der Indikator in Bezug auf den Erfolg oder die Erfolglosigkeit der optischen Behandlung gibt;
Kombinieren der gewichteten mindestens zwei Indikatoren zu einem gemeinsamen Indikatorwert, wobei der gemeinsame Indikatorwert den Erfolg oder die Erfolglosigkeit der optischen Behandlung zur Heilung oder Linderung der vorbestimmten Krankheit darstellt; und
Bestimmen, ob die Parameterwerte der optischen Behandlungsvorrichtung geändert werden müssen oder nicht, auf Basis der empfangenen ersten Information und des gemeinsamen Indikatorwerts.

**Revendications**

1. Appareil de traitement optique comprenant :

une interface (306) configurée pour recevoir des premières informations relatives aux paramètres d'un traitement pour l'amélioration de l'état mental ou physique d'au moins un utilisateur (102) ;
des éléments irradiants (108A, 108B) configurés pour être portés par l'utilisateur (102) dans les oreilles de l'utilisateur (102) et délivrer une énergie de radiation optique (106) pour éclairer un tissu optiquement sensible (104) de l'utilisateur (102) directement à travers un canal auditif externe (110A et 110B) de l'utilisateur (102) pour traiter ou soulager une maladie prédéterminée ;
dans lequel l'interface (306) est en outre configurée pour recevoir des deuxièmes informations comprenant au moins deux indicateurs différents (800A, 800B) indiquant si le traitement optique est ou non réussi, dans lequel les deuxièmes informations sont différentes des premières informations ;
et l'appareil (300) comprend en outre un processeur (302) configuré pour :

appliquer un coefficient de pondération individuel prédéterminé (804A, 804B) à chacun des deux indicateurs ou plus (800A, 800B) reçus ; dans lequel la valeur d'un coefficient de pondération donné est basée sur l'implication que l'indicateur donne relativement à la réussite ou à l'échec du traitement optique dans la guérison ou l'atténuation de la maladie prédéterminée ;
combiner les deux indicateurs pondérés ou plus en une valeur d'indicateur commune, dans lequel la valeur d'indicateur commune représente la réussite ou l'échec du traitement optique dans la guérison ou l'atténuation de la maladie prédéterminée ; et
déterminer si les valeurs de paramètres de l'appareil de traitement optique doivent être ou non modifiées, sur la base des premières informations reçues et de la valeur d'indicateur commune.

2. Appareil (300) selon la revendication 1, dans lequel le processeur (302) est en outre configuré pour :

analyser la valeur d'indicateur commune actuellement déterminée de manière à déterminer la modification par rapport à la valeur d'indicateur commune préalablement déterminée, et déterminer que le traitement optique est une réussite lorsque la modification par rapport à la valeur d'indicateur commune préalable est dans la direction positive, auquel cas aucune modification des valeurs de paramètres n'est nécessaire, ou déterminer que le traitement optique est une réussite lorsque la valeur d'indicateur commune dépasse au moins un seuil prédéterminé, auquel cas aucune modification des valeurs de paramètres n'est nécessaire.

3. Appareil (300) selon l'une quelconque des revendications 1 à 2, dans lequel les deux indicateurs ou plus sont obtenus d'au moins l'une des manières suivantes : entrés par l'utilisateur (102), téléchargés d'un dispositif électronique externe (310), et téléchargés d'un réseau (312).

4. Appareil (300) selon l'une quelconque des revendications 1 à 3, dans lequel au moins un indicateur (800A, 800B) des deux indicateurs ou plus comprend un indicateur lié à la voix de l'utilisateur (102) obtenu par un capteur externe porté par un utilisateur (102) durant la ou les périodes d'observation prédéterminées.

5. Appareil (300) selon la revendication 4, dans lequel l'indicateur lié à la parole est au moins l'un des suivants : le ton de la parole, la quantité de rire, la quantité de paroles agressives, et le rapport du monologue sur le dialogue.

6. Appareil (300) selon l'une quelconque des revendications 1 à 5, dans lequel au moins un indicateur (800A, 800B) des deux indicateurs ou plus comprend un indicateur lié à l'activité de l'utilisateur (102) et indique au moins l'un de ce qui suit durant la ou les périodes d'observation prédéterminées : la quantité d'exercice réalisée par un utilisateur (102), la différence de l'ampleur entre l'activité moyenne dans un état de sommeil et l'activité moyenne dans un état d'éveil, l'activité moyenne (612), la durée d'activité au-dessus d'un certain seuil, la quantité d'activité nocturne, le niveau maximal d'activité, un rythme circadien, la différence de l'ampleur entre le niveau d'activité le plus élevé et le niveau d'activité le plus bas (614), et la durée de sommeil (616).

7. Appareil (300) selon l'une quelconque des revendications 1 à 6, dans lequel l'interface (306) est en outre configurée pour :

recevoir des troisièmes informations comprenant au moins un facteur affectant la réussite du traitement optique, dans lequel les troisièmes informations sont différentes des premières informations et des deuxièmes informations ; et le processeur (302) est en outre configuré pour prendre en considération les troisièmes informations lors de la détermination du fait que les valeurs de paramètres de l'appareil de traitement optique doivent ou non être modifiées.

8. Appareil (300) selon la revendication 7, dans lequel le ou les facteurs comprennent au moins un facteur lié à l'environnement de l'utilisateur (102) durant au moins une période d'observation prédéterminée.

9. Appareil (300) selon l'une quelconque des revendications 7 à 8, dans lequel le facteur est lié à une ampleur de lumière ambiante (400, 404), et l'interface (306) est en outre configurée pour :

recevoir l'ampleur de lumière ambiante (400, 404) du réseau (312) lorsque le ou les emplacements de l'utilisateur (102) durant la ou les périodes d'observation prédéterminées sont connus, dans lequel le réseau (312) maintient une base de données météorologiques horodatée à jour pour chaque emplacement.

10. Appareil (300) selon l'une quelconque des revendications 7 à 8, dans lequel le facteur est lié à l'ampleur de lumière ambiante (400, 404), et l'interface (306) est en outre configurée pour :

obtenir l'ampleur de lumière ambiante (400, 404) d'une base de données personnelle, dans lequel la base de données personnelle est générée par l'utilisateur (102) et stocke les informations relatives à la quantité de lumière ambiante (400, 404) reçues durant la ou les périodes d'observation prédéterminées.

11. Appareil (300) selon l'une quelconque des revendications 7 à 10, dans lequel le ou les facteurs comprennent un facteur lié à au moins l'un de ce qui suit durant la ou les périodes d'observation prédéterminées : apport alimentaire de l'utilisateur (201), et médicaments pris par l'utilisateur (102).

12. Appareil (300) selon l'une quelconque des revendications 7 à 11, dans lequel le processeur (302) est en outre configuré pour :

accorder la priorité à l'implication des deuxièmes informations par rapport aux troisièmes informations, dans lequel les deuxièmes et les troisièmes informations impliquent l'opposé relativement à la modification des valeurs de paramètres de l'appareil de traitement optique.

13. Appareil (300) selon l'une quelconque des revendications 1 à 12, dans lequel le processeur (302) est en outre configuré pour :

modifier, ou informer l'utilisateur (102) de modifier, les valeurs de paramètres de l'appareil de traitement optique sur la base du résultat de la détermination, dans lequel la valeur d'au moins l'un des paramètres suivants est modifiée : au moins une durée (904) d'éclairage de l'utilisateur (102) avec l'énergie de rayonnement optique (106), l'intervalle (912) d'éclairage de l'utilisateur (102) avec l'énergie de rayonnement optique (106), la puissance (906) de l'énergie de rayonnement optique (106), et au moins un point dans le temps (910) lorsque l'éclairage à lieu.

14. Appareil (300) selon l'une quelconque des revendications 1 à 13, dans lequel au moins un indicateur (800A, 800B) des deux indicateurs ou plus comprend un indicateur indiquant la quantité d'exercice réalisée par un utilisateur (102) durant la ou les périodes d'observation prédéterminées.

15. Produit de programme informatique mis en oeuvre sur un support de distribution lisible par ordinateur et comprenant des instructions de programme qui, lorsqu'elles sont chargées dans un appareil selon l'une quelconque des revendications 1 à 14, entraînent l'exécution par l'appareil de ce qui suit :

obtention de premières informations liées aux paramètres d'un traitement pour améliorer l'état mental ou physique d'au moins un utilisateur (102) ;
obtention de deuxièmes informations comprenant au moins deux indicateurs (800A, 800B) indiquant si le traitement optique est ou non réussi, dans lequel les deuxièmes informations sont différentes des premières informations ;
application d'au moins un coefficient de pondération prédéterminé (804A, 804B) à chacun des deux indicateurs ou plus (800A, 800B) reçus, dans lequel la valeur d'un coefficient de pondération prédéterminé donné est basée sur l'implication que l'indicateur donne relativement à la réussite ou à l'échec du traitement optique ;
combinaison des deux indicateurs pondérés ou plus en une valeur d'indicateur commune, dans lequel la valeur d'indicateur commune représente la réussite ou l'échec du traitement optique dans la guérison ou l'atténuation de la maladie prédéterminée ; et détermination de la nécessité ou non de modifier les valeurs de paramètres de l'appareil de traitement optique, sur la base des premières informations reçues et de la valeur d'indicateur commune.

FIG. 1

FIG. 2

FIG. 3

FIG. 4

FIG. 5

FIG. 6

FIG. 7

| | | 804A | 806A | |
|---|---|---|---|---|
| 800A | 802A | | | |
| | VERY HIGH | 3 | 0 | |
| 1ST INDICATOR "RATIO OF MONOLOGUE AND DIALOGUE" | **HIGH** | **2** | 2 | |
| | LOW | 1 | 0 | |
| | VERY LOW | 0 | 0 | |
| 2ND INDICATOR "EXERCISE" | **A LOT** | **10** | 10 | |
| | SOME | 5 | 0 | |
| | A LITTLE | 2 | 0 | |
| | NONE | 0 | 0 | |
| 800B | 802B | 804B | 806B | |

X  =  =>

$$\sum_{i=1}^{8} = 12 > T$$

MODIFY
PARAMETERS
⇑
TREATMENT
FAILED
⇑
NO
⇑

YES
⇓
TREATMENT
SUCCEEDED
⇓
KEEP
PARAMETERS

## FIG. 8

## FIG. 9

FIG. 10